# EUROPÄISCHE PATENTANMELDUNG

(11) **EP 4 755 327 A1**
(43) Veröffentlichungstag der Anmeldung: **10.06.2026**
(21) Anmeldenummer: 24218038.8
(22) Anmeldetag: 06.12.2024
(51) Int. Cl.: A61B 18/08

(54) **ELEKTROCHIRURGISCHES INSTRUMENT**

(71) Anmelder: Erbe Elektromedizin GmbH, 72072 Tübingen (DE)
(72) Erfinder: HEIDER, Tanja, 72138 Kirchentellinsfurt (DE)
(74) Vertreter: Rüger Abel Patentanwälte PartGmbB

(57) **Zusammenfassung**

Die Erfindung betrifft ein elektrochirurgisches Instrument (10), insbesondere zum thermischen Schneiden von biologischem Gewebe eines Patienten. Das weist mindestens zwei Branchen (15, 16) auf, wobei mindestens eine der Branchen (15) dazu eingerichtet ist, relativ zu der anderen Branche (16) auf diese zu und von dieser weg bewegt zu werden. Mindestens eine der Branchen (15) weist ein thermisches Schneidelement (22) mit einem elektrisch leitfähigen Schneidleiter (36) und einem elektrisch leitfähigen Rückleiter (37) auf, die miteinander elektrisch verbunden sind. Das Schneidelement (22) ist derart angeordnet, dass der Schneidleiter (36) an einer der anderen Branche (16) zugewandten Seite der einen Branche (15) wenigstens teilweise zumindest thermisch frei liegt und der Rückleiter (37) von einem Isolierkörper 34 umgeben ist, wobei der Rückleiter (37) einen geringeren elektrischen Widerstand und vorzugsweise eine größere Wärmeleitfähigkeit als der Schneidleiter (36) aufweist, wodurch am Rückleiter (37) bei Bestromung des Schneidelements (22) eine wesentlich geringere Wärme als am Schneidleiter (36) erzeugt und der Rückleiter (37) zur Wärmeabfuhr verwendet werden kann.

## Beschreibung

Die Erfindung betrifft ein elektrochirurgisches Instrument, insbesondere zum thermischen Schneiden von biologischem Gewebe eines menschlichen oder tierischen Patienten sowie, gegebenenfalls zur Erzielung weiterer Gewebeeffekte.

Elektrochirurgische Instrumente mit thermischen Schneidelementen sind aus dem Stand der Technik in unterschiedlichen Ausführungen bekannt.

In der WO 2023/187737 A1 ist ein chirurgisches Instrument mit einer thermischen Schneidvorrichtung offenbart. Die thermische Schneidvorrichtung weist einen länglichen Träger mit einem proximalen und einem distalen Ende und einer Schneidkante auf, die entlang einer oberen Fläche desselben angeordnet ist. Außerdem weist die Schneidvorrichtung einen dielektrischen Isolator auf, der entlang mindestens einer Seite des Substrats angeordnet ist und sich mindestens teilweise entlang desselben vom proximalen zum distalen Ende erstreckt. Darüber hinaus weist die Schneidanordnung mindestens ein Widerstandselement auf, das zur Verbindung mit einer Energiequelle geeignet ist und in thermischer Verbindung mit dem Substrat angeordnet ist. Das mindestens eine Widerstandselement ist dabei derart angeordnet, dass es sich entlang des dielektrischen Isolators zu einem distalen Endabschnitt desselben erstreckt. Des Weiteren weist die Schneidanordnung ein Verkapselungsmaterial auf, das sowohl auf dem dielektrischen Isolator als auch auf dem mindestens einen Widerstandselement angeordnet ist. Das distale Ende des Substrats weist eine mechanische Schnittstelle auf, die dazu eingerichtet ist, mit einem Abschnitt der Branche in Eingriff zu kommen, um das Substrat an der Branche zu befestigen.

Weitere thermische Schneidelemente sind in WO 2023/187735 A1, US 2023/0363812 A1, WO 2023/187736 A1, US 2023/0310063 A1, EP 3 861 950 A1, EP 3 769 709 B1, US 2022/0378494 A1 und US 2023/0285064 A1 beschrieben.

In der US 2023/240740 A1 wird ein thermisches Schneidelement beschrieben, bei dem Heizelemente auf einen elektrisch isolierenden Träger aufgetragen sind. Die Heizelemente können über die Länge hinweg eine variierende Querschnittsfläche aufweisen. Außerdem weisen die Heizelemente verschiedene Abschnitte auf, die beispielsweise linear oder meanderförmig ausgestaltet sein können, um ein gewünschtes Temperaturprofil zu erzeugen.

In der EP 4 076 239 B1 wird ein Verfahren zur Herstellung eines thermischen Schneidelementes für ein chirurgisches Instrument beschrieben. Bei dem Verfahren wird auf zumindest einen Abschnitt eines Trägers eine Beschichtung mittels eines plasmaelektrolytischen Oxidationsverfahrens (*Plasma Electrolytic Oxidation*) aufgetragen. Auf diese Beschichtung wird dann ein Heizelement aufgetragen.

Alle im Stand der Technik vorzufindenden Heizelemente weisen Trägermaterialien mit einer vergleichsweise hohen Wärmeleitfähigkeit auf, so dass sich die gesamte Branche auch an unerwünschten Stellen, wie z.B. der Außenseite der Branche, erwärmen kann. Dies kann für den Chirurgen zu einer Erschwernis des Eingriffs führen, da die Instrumentenbranche - insbesondere bei längeren Eingriffen - an unerwünschten Stellen im Operationsfeld am Gewebe des Patienten thermisch anhaften und dort das Gewebe ungewollt schädigen kann, was mit einer verlängerten Rekonvaleszenzzeit des Patienten nach dem Eingriff einhergehen kann.

Davon ausgehend liegt der vorliegenden Erfindung die Aufgabe zugrunde, ein verbessertes elektrochirurgisches Instrument zum thermischen Schneiden von biologischem Gewebe bereitzustellen. Im Idealfalle sollen sich die Branchen des Instruments während seiner Anwendung vorzugsweise nur gering oder überhaupt nicht erwärmen.

Diese Aufgabe wird mit dem elektrochirurgischen Instrument nach Anspruch 1 gelöst:
Das erfindungsgemäße chirurgische Instrument ist insbesondere zum thermischen Schneiden von biologischem Gewebe eines Patienten eingerichtet. Das Instrument weist mindestens zwei Branchen auf, wobei mindestens eine der Branchen dazu eingerichtet ist, relativ zu der anderen Branche auf diese zu und von dieser weg bewegt zu werden. Beispielsweise ist eine der Branchen mittels einer Schwenkeinrichtung um eine Schwenkachse schwenkbar eingerichtet. Es können auch beide Branchen zangenartig aufeinander zu und voneinander weg bewegbar ausgebildet sein. Mindestens eine der Branchen weist ein thermisches Schneidelement mit einem elektrisch leitfähigen Schneidleiter und einem elektrisch leitfähigen Rückleiter auf. Das Schneidelement ist derart angeordnet, dass der Schneidleiter an einer der anderen Branche zugewandten Seite der einen Branche zumindest thermisch frei liegt und der Rückleiter von einem Isolierkörper (vollständig) umgeben ist. Der Schneidleiter kann metallisch blank ausgebildet oder mit einem thermisch leitfähigen, elektrisch isolierenden Überzug überdeckt sein. Beispielsweise kann der Überzug hydrophobe Eigenschaften aufweisen, wodurch ein Anhaften des Schneidleiters an dem biologischen Gewebe des Patienten verhindert werden kann.

Der Schneidleiter und der Rückleiter können mit einer Stromquelle verbindbar sein und von dieser gespeist werden. Eine solche Stromquelle kann eine Gleichstromquelle aber auch eine Wechselstromquelle, beispielsweise eine Hochfrequenzquelle (HF-Quelle) sein.

Eine Besonderheit der vorliegenden Erfindung besteht darin, dass der Rückleiter einen geringeren elektrischen Widerstand und vorzugsweise eine höhere Wärmeleitfähigkeit als der Schneidleiter aufweist. Der Isolierkörper ist elektrisch und thermisch isolierend ausgebildet, so dass bei Bestromung des Schneidelements Wärme ganz vorwiegend am Schneidleiter erzeugt wird und dort insbesondere von dem Isolierkörper am rückwärtigen Abfluss gehindert wird. Der Isolierkörper kann beispielsweise aus Silikon bestehen. Weil der Rückleiter einen geringeren elektrischen Widerstand als der Schneidleiter aufweist, kann dort bei Bestromung des Schneidelements eine wesentlich geringere Wärme als am Schneidleiter erzeugt werden. Der Rückleiter weist insbesondere einen Kern auf (den Rückleiterkern), der mit einem Überzug überzogen ist. Durch den Überzug können die Eigenschaften des Rückleiter, insbesondere der elektrische Widerstand und die Wärmeleitfähigkeit des Rückleiters, beeinflusst werden. Vorzugsweise kann der elektrische Widerstand des Rückleiters mittels des Überzugs geringer als der elektrische Widerstand des Rückleiterkerns ausgebildet sein. Der Rückleiterkern besteht vorzugsweise aus demselben Material wie der Schneidleiter.

Der Schneidleiter und der Rückleiter sind miteinander elektrisch und körperlich verbunden. Der Rückleiter weist insbesondere in Leiterrichtung - also in und entgegen der Richtung, in der Strom bei Benutzung des Schneidelements fließt - eine größere Wärmeleitfähigkeit als der Schneidleiter auf. Der Überzug des Rückleiters weist insbesondere eine größere Wärmeleitfähigkeit als das Grundmaterial des Rückleiterkerns auf. Der Überzug führt dazu, dass der elektrische Widerstand des Rückleiters, d.h. des Gesamtverbunds aus Rückleiterkern und Überzug, geringer als der elektrische Widerstand des Schneidleiters ist. Außerdem weist der Rückleiter aufgrund des Überzugs eine höhere Wärmeleitfähigkeit als der Rückleiterkern ohne Überzug auf. Durch die hohe Wärmeleitfähigkeit des Rückleiters kann die am Rückleiter erzeugte Wärme abgeführt werden, was besonders vorteilhaft ist, wenn der Rückleiter einen besonders kleinen Querschnitt aufweist. Es kann so verhindert werden, dass der Rückleiter (Gesamtverbund aus Rückleiterkern und Überzug) überhitzt. Die Branche kann so besonders schlank und besonders flach ausgestaltet werden, ohne dass beim Schneiden lokal zu viel Wärme von dem Rückleiter zu dem Tragteil der Branche und damit an die Außenseite der Branche transportiert wird.

Der Schneidleiter und der Rückleiter sind vorzugsweise ein einstückiger Körper. Dieser ist beispielsweise zumindest im Wesentlichen U-förmig ausgestaltet, wobei der Schneidleiter und der Rückleiter jeweils einen Schenkel des U-förmigen Schneidelements bilden können.

Die Branche weist insbesondere ein längliches Tragteil auf, welches sich von einem proximalen Bereich, in welchem eine Scharniereinrichtung zur schwenkbaren Lagerung der anderen Branche angeordnet sein kann, zu einem distalen Endbereich erstreckt. Das Tragteil kann einen Aufnahmeraum umgrenzen, in dem das Schneidelement vorzugsweise vertikal angeordnet ist. Dabei ist der Schneidleiter oberhalb des Rückleiters angeordnet.

Der Schneidleiter und der Rückleiter bestehen vorzugsweise aus ein- und demselben Grundmaterial. Auf diesem können an verschiedenen Stellen verschiedene Überzüge vorgesehen sein; einzelne Stellen, z.B. der Schneidleiter, Anschlussbereiche oder dergleichen, können auch frei von Überzügen sein.

Das U-förmige Schneidelement ist vorzugsweise zum proximalen Bereich der Branche hin offen. An dem proximalen Ende können Anschlussbereiche oder -elemente mit dem Schneidleiter und dem Rückleiter elektrisch verbunden sein, mittels derer das Schneidelement mit Zuleitungen verbunden sein kann. Über diese kann das Schneidelement mit der Stromquelle verbunden und von dieser gespeist werden. Durch die einstückige Ausgestaltung des Schneidelements ist insbesondere am distalen Endbereich der Branche, in welchem der Schneidleiter mit dem Rückleiter körperlich - also elektrisch und thermisch - verbunden ist, keine Verbindungsnaht, z.B. Schweiß- oder Lötnaht, notwendig. Das Risiko von Leiterbrüchen zwischen dem Schneidleiter und dem Rückleiter bei Benutzung des Instruments und der damit verbundenen mechanischen Beanspruchung des Schneidelements ist signifikant reduziert.

Der Rückleiterkern ist vorzugsweise zumindest teilweise mit einem sich in Längsrichtung erstreckenden Überzug versehen. Dieser Überzug deckt vorzugsweise den gesamten Außenumfang des Rückleiterkerns ab. Der Überzug ermöglicht es, den Rückleiterkern und den Schneidleiter aus einem Material sowie einteilig herzustellen, wodurch die Notwendigkeit eines zusätzlichen Elements zum Verknüpfen der beiden Leiter (Schneid- und Rückleiter) entfällt. Es wird bevorzugt, dass der gesamte Rückleiter mit dem Überzug versehen ist, wodurch die an dem Rückleiter erzeugte Wärme mittels des Überzugs des Rückleiters großflächig von diesem abgeleitet werden kann.

Der Überzug besteht vorzugsweise aus einem Material, das einen niedrigeren spezifischen elektrischen Widerstand als das Grundmaterial des Schneidleiters aufweist. Das Material des Überzugs weist vorzugsweise zusätzlich eine höhere spezifische Wärmeleitfähigkeit als das Grundmaterial des Schneidleiters auf. Der Schneidleiter und der Rückleiter können aus demselben Material bestehen, wie z.B. aus Edelstahl, aus einer Nickel-Basis-Legierung, die beispielsweise Eisen, Molybdän, Niob, Kobalt, Mangan, Kupfer, Aluminium, Titan, Silizium, Kohlenstoff, Schwefel, Phosphor, und Bohr enthalten kann, oder aus einer Eisen-Chrom-Aluminium-Legierung. Der Überzug besteht beispielsweise aus Kupfer, Silber und/oder Aluminium. Die spezifische Wärmeleitfähigkeit des Überzugs ist insbesondere größer als 200 W/(m·K).

Insbesondere weist der Schneidleiter eine größere Querschnittsfläche und einen höheren spezifischen Widerstand als der Rückleiter auf, wodurch der elektrische Widerstand des Schneidleiters größer, vorzugsweise deutlich größer als der elektrische Widerstand des mit dem Überzug versehenen Rückleiters mit geringerem elektrischem Widerstand und hoher Wärmeleitfähigkeit ist. Hierdurch kann die in dem Schneidelement erzeugte Wärme vorrangig am Schneidleiter erzeugt werden, wobei der Rückleiter vergleichsweise kalt bleibt und zur Wärmeabfuhr dient.

In einem Randbereich der Branchen weisen diese vorzugsweise jeweils mindestens eine Versiegelungselektrode auf, die zu dem Schneidleiter beabstandet abgeordnet ist. Insbesondere ist der Schneidleiter, zumindest an seinen Seitenflanken, zwischen dem distalen und dem proximalen Ende der Branche von der Versiegelungselektrode umschlossen.

Die Versiegelungselektroden sind insbesondere dazu eingerichtet an eine Stromquelle, beispielsweise an eine HF-Stromquelle, angeschlossen zu werden. Die Versiegelungselektroden und das Schneidelement können mit einer (einzigen) HF-Stromquelle versorgt werden.

Die Versiegelungselektroden und der Schneidleiter können aus einer gemeinsamen Speisequelle, z.B. HF-Stromquelle gespeist werden. Ein in dem Instrument vorgesehenes Netzwerk, z.B. ein Transformator, kann dabei dazu genutzt werden, aus der Spannung der Speisequelle die unterschiedlichen zum Betrieb der Versiegelungselektroden und des Schneidelements erforderlichen Spannungen bereit zu stellen. Das Netzwerk (der Transformator) kann Teil des Instruments oder des speisenden Generators sein. Es ist aber auch möglich, die Versiegelungselektroden und das Schneidelement mit jeweils eigenen (getrennten) HF-Stromquellen zu versorgen.

Insbesondere weist auch die andere (zweite) Branche in einem Randbereich mindestens eine Versiegelungselektrode auf. Die Versiegelungselektrode ist vorzugsweise identisch zu der Versiegelungselektrode der einen (ersten) Branche ausgestaltet, so dass beide bei geschlossenen Branchen übereinander, aber zueinander beabstandet, angeordnet sind. Die beiden Versiegelungselektroden sind an die Pole eines speisenden Generators angeschlossen. In der zweiten Branche ist ein elastischer Gegendruckkörper angeordnet, gegen den der Schneidleiter beim Schließen der Branchen anläuft, um es elastisch etwas zu verformen.

Vorzugsweise besteht der Isolierkörper (und auch der Gegendruckkörper) aus einem Material, das eine niedrigere Wärmeleitfähigkeit als der Schneidleiter aufweist, wodurch der Schneidleiter von dem Rückleiter thermisch weitgehend isoliert sein kann. Die von dem Schneidleiter erzeugte Wärme kann so auf einen schmalen Streifen des biologischen Materials konzentriert und dort wirksam werden.

An dem Schneidleiter kann mindestens ein Befestigungsvorsprung ausgebildet sein, der von dem Isolierkörper umgeben ist. Vorzugsweise weist der Befestigungsvorsprung mindestens einen Hinterschneidungsbereich auf. Der Befestigungsvorsprung ist insbesondere vollständig von dem Isolierkörper umgeben, wodurch verhindert werden kann, dass sich der Schneidleiter von dem Isolierkörper mechanisch löst oder herausgezogen werden kann, beispielsweise wenn der Schneidleiter am biologischen Gewebe anhaftet und das Instrument weiterbewegt wird.

Zwischen dem Schneidleiter und dem Rückleiter kann wenigstens ein Abstandselement angeordnet sein. Das Abstandselement kann beispielsweise aus einem elektrisch und thermisch isolierenden Material bestehen. Beispielsweise aus Keramik wie z.B. ZrO₂ oder AlO₂. Das Material, aus dem das Abstandselement besteht, kann insbesondere Temperaturen von mehr als 350 °C standhalten.

In einer Ausführungsform kann das Abstandselement z.B. eine (ebene) Oberseite und eine (ebene) Unterseite aufweisen. Vorzugsweise liegt das Abstandselement mit seiner Oberseite an dem Schneidleiter und/oder mit seiner Unterseite an dem Rückleiter an. Hierdurch kann verhindert werden, dass sich der Schneidleiter bei mechanischer Beanspruchung durchbiegt. Der Isolierkörper weist, insbesondere wenn er aus Silikon besteht, eine gewisse elastische Verformbarkeit auf. Durch das Abstandselement kann sich der Schneidleiter an dem Rückleiter abstützen, wodurch eine Verformung des Schneidleiters verhindert werden kann.

In einer weiteren Ausführungsform sind zwischen dem Schneidleiter und dem Rückleiter mehrere, insbesondere mindestens drei, Abstandselemente angeordnet. Jedes der Abstandselemente kann, beispielsweise an seiner Unterseite, eine Rückleiteraussparung aufweisen, von der der Rückleiter derart aufgenommen sein kann, dass dieser von dem Abstandselement zumindest teilweise umschlossen eingefasst ist, wodurch die Abstandselemente dazu beitragen, dass aus Schneidleiter und Rückleiter bestehende Schneidelement in der einen Branche zu positionieren und zu fixieren. Vorzugsweise ist die Rückleiteraussparung zu einer Seite hin geöffnet. Dies vereinfacht die Montage des Schneidelements an der Branche. Vorzugsweise sind die mehreren, insbesondere mindestens drei, Abstandelemente derart angeordnet, dass die Seite abwechselt, zu der die Rückleiteraussparungen hin geöffnet sind.

Vorzugsweise weisen die mehreren Abstandselemente je eine Auflagefläche auf, auf der der Schneidleiter aufliegt. Es liegt insbesondere die Unterseite des Schneidleiters auf der Auflagefläche auf. Das Abstandselement weist wenigstens einen seitlichen Anschlag auf, der von der Auflagefläche vorsteht und an dem der Schneidleiter seitlich anliegt. Vorzugsweise sind die mehreren Abstandselemente derart angeordnet, dass die Seite abwechselt, an der der wenigstens eine Anschlag von der Auflagefläche vorsteht.

Es wird bevorzugt, dass die andere (zweite) Branche den Gegendruckkörper aufweist, der an einer der einen (ersten) Branche zugewandten Seite angeordnet ist und dort eine (ebene) Gegendruckfläche für den Schneidleiter der einen (ersten) Branche definiert. Der Gegendruckkörper ist vorzugsweise elektrisch und thermisch isolierend ausgebildet. Der Gegendruckkörper weist vorzugsweise eine gewisse Elastizität auf, so dass sich der Gegendruckkörper leicht verformen kann, wenn Teile des Schneidelements in ihn eindringen. Durch die Elastizität sorgt der Gegendruckkörper dafür, dass das Gewebe beim Schneiden an dem Schneidleiter anliegt. Der Schneidleiter steht insbesondere von der durch den Isolierkörper gebildeten Oberfläche in Richtung der anderen Branche hin vor. Beispielsweise kann der Schneidleiter um 0,10mm, 0,15 mm, oder mehr vorstehen.

Insbesondere kann der Gegendruckkörper dazu eingerichtet sein, dass der Schneidleiter bei geschlossenen Branchen entlang des (gesamten) Schneidleiters, vorzugsweise mit einer gleichmäßigen Tiefe, in ihn eindringen kann.

Weitere Einzelheiten vorteilhafter Ausführungsform der Erfindung ergeben sich aus den Unteransprüchen aus der Zeichnung oder aus der Beschreibung. Es zeigen:
Figur 1 ein Beispiel des erfindungsgemäßen elektrochirurgischen Instruments in einer perspektivischen Ansicht;
Figur 2 ein Beispiel für das Werkzeug des Instruments am distalen Ende des Instruments in einer Detailansicht;
Figur 3 ein Beispiel der ersten Branche des Instruments in der Draufsicht;
Figur 4 ein Beispiel für die zweite Branche des Instruments in der Draufsicht;
Figur 5 ein Beispiel für das Schneidelement mit einem Teil des Isolierkörpers in einer Detailansicht;
Figur 6 ein Beispiel für die beiden Branchen des Instruments in geschlossenem Zustand im Längsschnitt;
Figur 7 ein Beispiel für die beiden Branchen des Instruments in geschlossenem Zustand im Querschnitt;
Figur 8 ein weiteres Beispiel für die beiden Branchen des Instruments in geschlossenem Zustand im Querschnitt;
Figur 9 ein weiteres Beispiel der ersten Branchen des Instruments im Längsschnitt;
Figur 10 eine Detailansicht des Schneidelements mit den Abstandselementen von einer Seite;
Figur 11 eine Detailansicht des Schneidelements mit den Abstandselementen von der anderen Seite; sowie
Figur 12 die untere Branche des Instruments im Querschnitt.

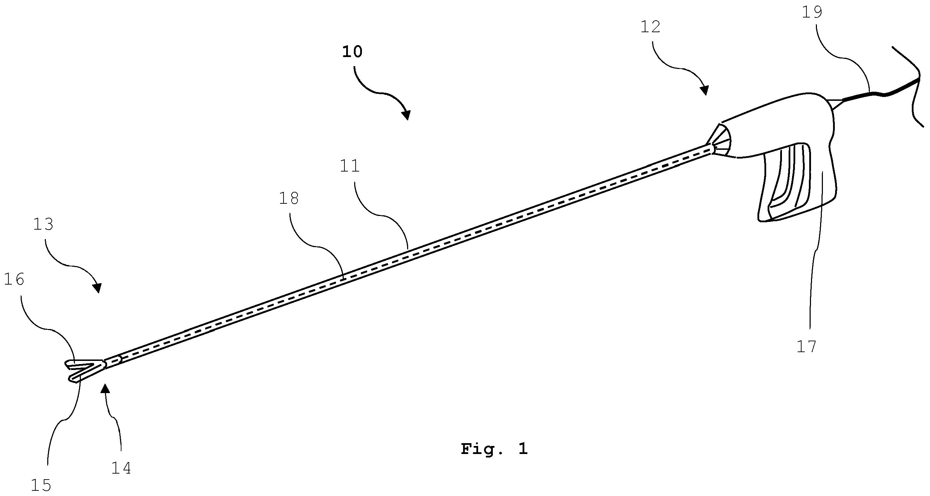

Figur 1 zeigt ein Beispiel des elektrochirurgischen Instruments 10, das zum thermischen Schneiden von biologischem Gewebe eines Patienten eingerichtet ist. Das Instrument 10 weist einen länglichen Schaft 11 auf, der sich von einem proximalen Ende 12 des Instruments 10 zu einem distalen Ende 13 des Instruments 10 erstreckt. An dem distalen Ende 13 des elektrochirurgischen Instruments 10 ist ein Werkzeug 14 angeordnet, welches eine erste Branche 15 und eine zweite Branche 16 aufweist, bei der mindestens eine der beiden Branchen 15, 16 relativ zu der anderen bewegbar ist.

An dem proximalen Ende 12 des elektrochirurgischen Instruments 10 ist ein Handgriff 17 zum Betätigen des Werkzeugs 14 angeordnet. In dem Schaft 11 können Kanäle angeordnet sein, in denen elektrische von dem proximalen Ende 12 zum distalen Ende 13 des Instruments 10 laufende Zuleitungen 18 untergebracht sind, um das Werkzeug 14 mit darin angeordneten Schneid- und Versiegelungselektroden elektrisch zu versorgen. Darüber hinaus können in dem Schaft 11 ein oder mehrere Steuerdrähte vorgesehen sein, von denen mindestens einer mit mindestens einer der beiden Branchen 15, 16 verbunden ist, um diese zu öffnen und zu schließen. An dem Handgriff 17 des Instruments 10 ist eine Instrumentenzuleitung 19 angebracht, mit welcher das elektrochirurgische Instrument 10 an ein Versorgungsgerät zur Versorgung des Instruments 10 mit Spannung, Strom oder anderen Medien angeschlossen werden kann.

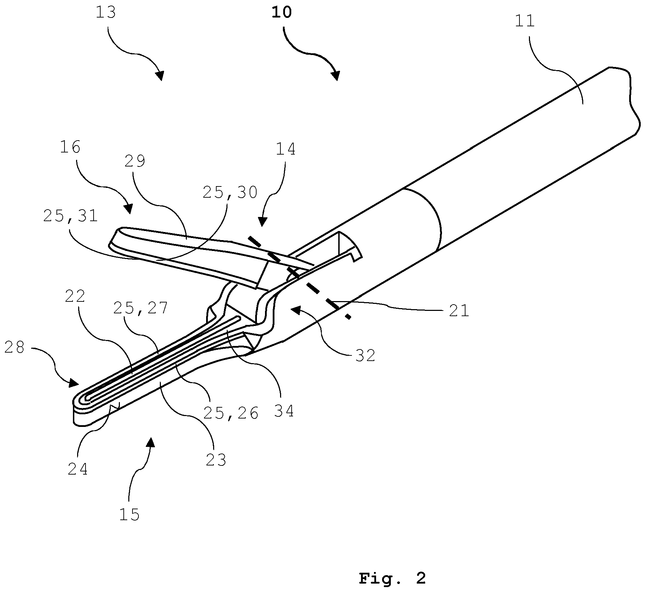

Figur 2 zeigt eine Detailansicht des Werkzeugs 14, das am distalen Ende 13 des Schafts 11 angeordnet ist. Die beiden Branchen 15, 16 können nach Art einer Zange geöffnet und geschlossen werden. Dazu ist mindestens eine der beiden Branchen 15, 16 - im vorliegenden Ausführungsbeispiel ist es die zweite Branche 16 - mittels einer durch ihre Schwenkachse 21 angedeutete Scharniereinrichtung gegen die erste Branche 15 schwenkbar, die feststehend angeordnet ist. Alternativ können auch beide Branchen 15, 16 aufeinander zu- und voneinander weg schwenkbar angeordnet sein. Die Scharniereinrichtung kann durch ein oder zwei Schwenklager, eine Kulissenführung, ein Federgelenk oder dergleichen gebildet sein.

In mindestens einer der beiden Branchen 15, 16 ist ein thermisches Schneidelement 22 zum thermischen Schneiden von biologischem Gewebe angeordnet. Die Branchen 15, 16 sind in Figur 2 besonders schmal und schlank ausgestaltet und zumindest im Wesentlichen gerade ausgebildet. Anders als in Figur 2 gezeigt, können die Branchen 15, 16 auch eine leichte Krümmung aufweisen, wodurch mit dem elektrochirurgischen Instrument, beispielsweise das Präparieren von Organen und anderen biologischen Geweben möglich ist. Das elektrochirurgische Instrument 10 dient insbesondere zum Schneiden, Trennen, Schließen und Versiegeln von Gefäßen, beispielsweise Blutgefäßen.

Die erste Branche 15 ist durch ein steifes, beispielsweise aus Metall gebildetes Tragteil 23 gebildet, das an seiner Außenseite 24 eine elektrische Isolation tragen kann. Alternativ kann das Tragteil 23 auch teilweise oder auch vollkommen aus einem mechanisch stabilen, weniger oder nicht flexiblen und elektrisch isolierenden Kunststoff bestehen. Auch kann das Tragteil 23 ein Verbundteil sein und zum Beispiel aus einem mit Kunststoff umspritzten Metall-Inlay gebildet sein.

Entlang seiner beiden Randbereiche 25 ist das Tragteil 23 der ersten Branche 15 mit Versiegelungselektroden 26 und 27 versehen, die über nicht weiter dargestellte Leitungen mit einem elektrischen Generator verbindbar sind. Die beiden Versiegelungselektroden 26 und 27 können in einem distalen Endbereich 28 der ersten Branche 15 untereinander körperlich und elektrisch verbunden sein, wie dies in Figur 2 dargestellt ist. Alternativ können jedoch auch gesonderte Versiegelungselektroden 26 und 27 vorgesehen sein, die auf gleichen oder unterschiedlichen Potenzialen liegen und in dem distalen Endbereich 28 körperlich nicht verbunden sind.

Die zweite Branche 16 weist ebenfalls ein Tragteil 29 auf, das wiederum aus Metall oder auch aus Kunststoff oder einem Metall-Kunststoff-Verbundteil gebildet sein kann. An seinem äußeren Rand trägt das Tragteil 29 die Versiegelungselektroden 30, 31, die sich von dem scharniernahen Bereich 32 bis zu dem distalen Endbereich 28 erstrecken. Die Kontur des Tragteils 29 der zweiten Branche 16 ist deckungsgleich zu der Kontur der ersten Branche 15. Sind die Branchen geschlossen, deckt sich die Versiegelungselektrode 26 mit der Versiegelungselektrode 30. Zudem deckt sich die Versiegelungselektrode 27 mit der Versiegelungselektrode 31. Außerdem können die Versiegelungselektroden 30, 31 in dem distalen Endbereich 28 der zweiten Branche 16 untereinander elektrisch und körperlich verbunden sein.

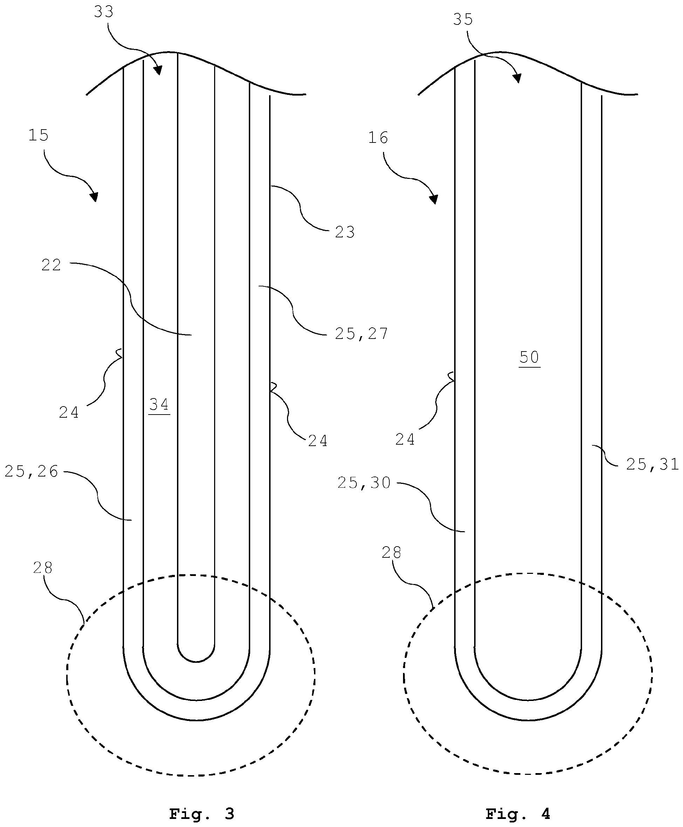

Figur 3 zeigt eine Draufsicht auf die erste Branche 15. Das Tragteil 23 der ersten Branche 15 weist eine Nut 33 auf, in der sich das Schneidelement 22 befindet. Das Schneidelement 22 ist von einem Isolierkörper 34 derart umgeben, dass die Oberseite des Schneidelements 22, an die der Schneidleiter 36 angeordnet ist, aus dem Isolierkörper 34 hervorragt.

Figur 4 zeigt eine Draufsicht der zweiten Branche 16. In diesem Ausführungsbeispiel ist in der zweiten Branche 16 kein Schneidelement 22 angeordnet. Alternativ kann auch in der zweiten Branche 16 ein Schneidelement 22 wie bei der ersten Branche 15 angeordnet sein. In dem in Figur 4 gezeigten Beispiel weist das Tragteil 29 der zweiten Branche 16 eine Nut 35 auf, in der ein Gegendruckkörper 50 angeordnet ist.

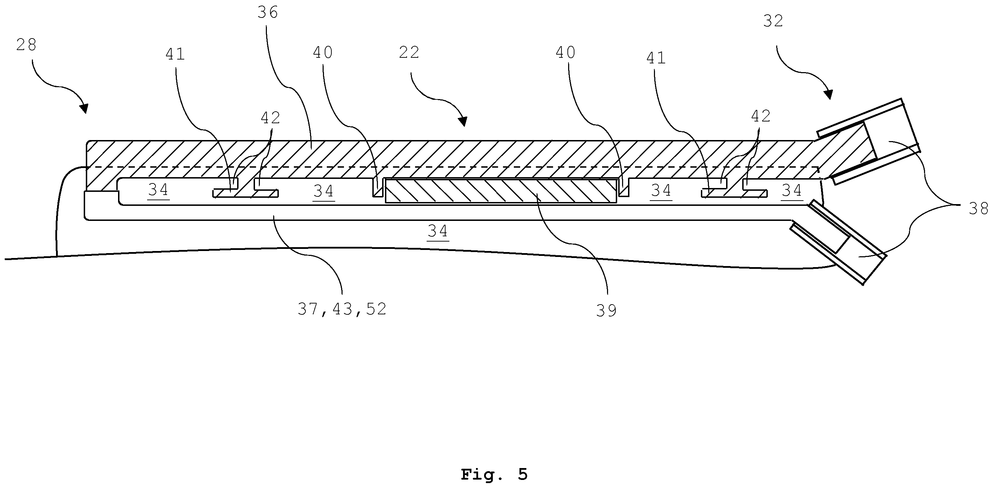

Figur 5 zeigt das Schneidelement 22 im Längsschnitt. Das Schneidelement 22 weist einen Schneidleiter 36 und einen Rückleiter 37 auf. Der Schneidleiter 36 erstreckt sich von dem scharniernahen Bereich 32 bis hin zum distalen Endbereich 28. Im scharniernahen Bereich 32 sind der Schneidleiter 36 und der Rückleiter 37 elektrisch kontaktierbar, beispielsweise form- und/oder reibschlüssig. Beispielsweise können der Schneidleiter 36 und der Rückleiter 37 mit einem Silberdraht anzuklemmen, ohne dass zusätzliche Anschlusselemente erforderlich sind. In dem in Figur 5 gezeigten Beispiel ist an dem Schneidleiter 36 ein Anschlusselement 38 angebracht. Bei dem Anschlusselement 38 kann es sich um eine Anschlusshülse, beispielsweise eine Crimphülse oder dergleichen handeln. Der Rückleiter 37 erstreckt sich von dem distalen Endbereich 28 bis hin zum scharniernahen Bereich 32.

Der Rückleiter 37 ist unterhalb des Schneidleiters 36 angeordnet. In dem distalen Endbereich 28 sind der Schneidleiter 36 und der Rückleiter 37 elektrisch und körperlich verbunden. In dem scharniernahen Bereich 32 weist der Rückleiter 37 in dem in Figur 5 gezeigten Beispiel ebenfalls ein Anschlusselement 38 auf. Über die Anschlusselemente 38 können der Schneidleiter 36 und der Rückleiter 37 mit einer Stromquelle verbunden werden.

Bei der Stromquelle kann es sich um dieselbe Stromquelle handeln, die auch für die Versiegelungselektronen verwendet wird, wobei zwischen der Stromquelle und dem Schneidelement ein Anpassnetzwerk angeordnet sein kann. Das Anpassnetzwerk kann dazu eingerichtet sein, die von der Stromquelle gelieferte elektrische Leistung auf die Versiegelungselektroden und das Schneidelement aufzuteilen und die gelieferte Spannung an die benötigte Spannung anzupassen. Gleiches gilt für den gelieferten Strom und den benötigten Strom. Das Schneidelement 22 kann jedoch auch an eine gesonderte Stromquelle angeschlossen werden.

Zwischen dem Schneidleiter 36 und dem Rückleiter 37 ist ein Abstandselement 39 angeordnet, das aus einem thermisch isolierenden und elektrisch isolierenden Material besteht. Das Abstandselement 39 ist zumindest im Wesentlichen eben ausgebildet und liegt an seiner Oberseite an der Unterseite des Schneidleiters 36 und mit seiner Unterseite an der Oberseite des Rückleiters 37 an. Das Abstandselement 39 dient dazu, dass sich der Schneidleiter 36 bei mechanischer Beanspruchung nicht durchbiegt, sondern sich vielmehr an dem Rückleiter 37 über das Abstandselement 39 abstützt. Das Abstandselement 39 ist zwischen zwei Positioniervorsprüngen 40 angeordnet, die an der Unterseite des Schneidleiters 36 in Richtung des Rückleiters 37 vorstehen, diesen allerdings nicht berühren. An der dem Rückleiter 37 zugewandten Seite des Schneidleiters 36 sind außerdem gemäß Figur 5 zwei Befestigungsvorsprünge 41 angeordnet, die von dem Schneidleiter 36 in Richtung des Rückleiters 37 vorstehen. Die Befestigungsvorsprünge 41 weisen Hinterschneidungsabschnitte 42 auf.

Der Rückleiter 37 weist einen Kern 52 auf, der von einen Überzug 43 umgeben ist, so dass der Rückleiter 37 einen geringeren elektrischen Widerstand als der Schneidleiter 36 aufweist. Außerdem weist der Rückleiter 37 in Stromflussrichtung eine größere Wärmeleitfähigkeit als der Schneidleiter 36 auf.

Der Überzug 43 bedeckt den Kern 52 des Rückleiters 37 umfangsseitig vorzugsweise vollständig. In dem in Figur 5 gezeigten Beispiel erstreckt sich der Überzug 43 von dem distalen Endbereich 28 bis hin zum scharniernahen Bereich 32. Anders als in Figur 5 gezeigt, kann sich der Überzug 43 auch nur in einem Abschnitt des Kerns 52 des Rückleiters 37 befinden, der sich vorzugsweise an den distalen Endbereich 28 angrenzt, an dem der Schneidleiter 36 mit dem Rückleiter 37 elektrisch und körperlich verbunden ist. Der Rückleiter 37 und auch ein Teil des Schneidleiters 36 ist von dem Isolierkörper 34 vorzugsweise derart eingebettet, dass nur ein oberer Teil des Schneidleiters 36 aus dem Isolierkörper 34 herausschaut.

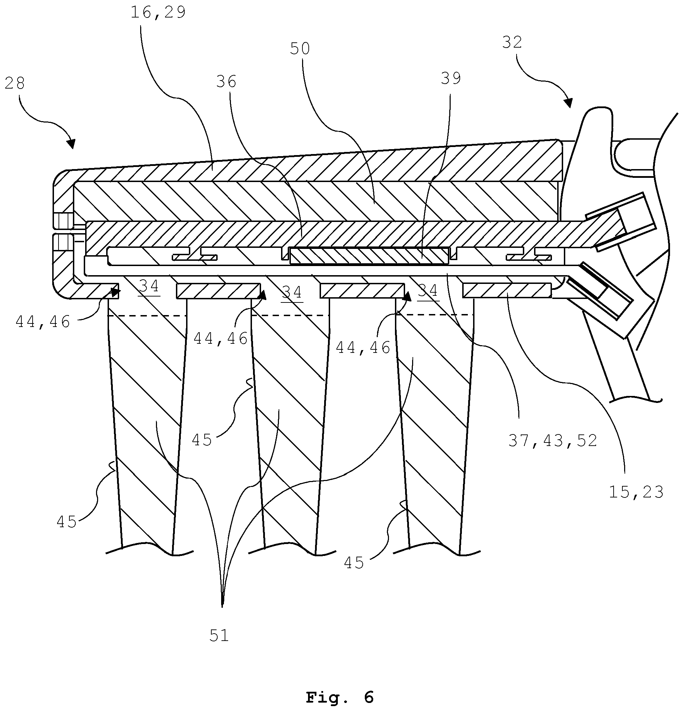

In Figur 6 ist das Werkzeug 14 in einem seitlichen Längsschnitt dargestellt. Das Schneidelement 22 ist von dem Isolierkörper 34 derart eingebettet, dass der Rückleiter 37 vollständig von dem Isolierkörper 34 umgeben ist. Der Isolierkörper 34 weist an seiner Unterseite mehrere Isolierkörperfüße 51 auf, die jeweils einen Hinterschneidungsbereich 44 aufweisen. Die Isolierkörperfüße 51 sind an den Stellen angeordnet, in denen in dem Tragteil 23 der ersten Branche 15 Öffnungen 46 vorgesehen sind.

Zur Montage des Schneidelements 22 wird dieses mit dem Isolierkörper 34 umspritzt. Die Isolierkörperfüße 51 weisen außerdem einen sich verjüngenden Abschnitt 45 auf, der dazu dienen kann, den Isolierkörper 34 an den Isolierkörperfüße 51 durch die Öffnungen 46 im Tragteil 23 der ersten Branche 15 hindurchzuziehen, bis der Hinterschneidungsbereich 44 in der Öffnung 46 sitzt. Durch den Hinterschneidungsbereich 44 sitzt der Isolierkörper 34 samt Schneidelement 22 formschlüssig im Tragteil 23 der ersten Branche 15. Nach dem Einsetzen des Isolierkörpers 34 in das Tragteil 23 können die Isolierkörperfüße 51 abgetrennt werden.

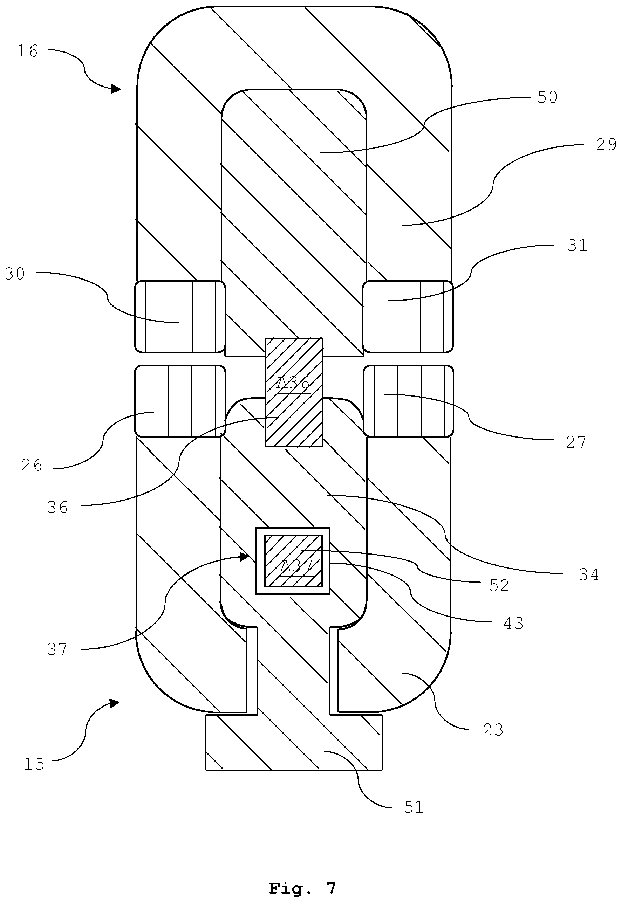

Figur 7 zeigt einen Querschnitt durch das Werkzeug 14 des Instruments 10. Insoweit gilt das zuvor gesagte unter Berufung auf die bereits eingeführten Bezugszeichen. Das Werkzeug 14 ist mit geschlossenen Branchen 15, 16 abgebildet. Der Schneidleiter 36 überschneidet sich in dem geschlossenen Zustand der Branchen 15, 16 zumindest teilweise mit dem Gegendruckkörper 50. Der Gegendruckkörper 50 dient dazu, sicherzustellen, dass das zu schneidende Gewebe des Patienten stets an dem Schneidleiter 36 anliegt, um einen sauberen Schnitt zu ermöglichen.

Darüber hinaus ist die Querschnittsfläche A36 des Schneidleiters 36 größer als die Querschnittsfläche A37 des Rückleiters 37. In dem in Figur 7 gezeigten Beispiel ist die Querschnittsfläche A37 des Rückleiters 37 in etwa halb so groß wie die Querschnittsfläche A36 des Schneidleiters 36. In dem in Figur 7 gezeigten Beispiel ist der Rückleiter 37 außerdem entlang seines gesamten Umfangs mit einem Überzug 43 beschichtet. Die niedrigere Querschnittsfläche A37 des Rückleiters 37 führt zunächst dazu, dass der elektrische Widerstand des Rückleiters 37 ohne Überzug größer ist als der des Schneidleiters. Weil der Überzug 43 jedoch aus besonders niederohmigem Material besteht, ist der elektrische Widerstand des Rückleiters 37 trotz seines geringeren Querschnitts A37 kleiner als der elektrische Widerstand des Schneidleiters 36. Beispielsweise beträgt eine Breite des Kerns 52 des Rückleiters 37 zwischen 0,2 und 0,5 mm und eine Höhe beträgt zwischen 0,2 und 0,5 mm. Die Überzugsdicke des Überzugs 43 beträgt z.B. zwischen 0,02 mm und 0,08 mm. Die Breite des Schneidleiters 36 beträgt ebenfalls zwischen 0,2 mm und 0,5 mm, während die Höhe des Schneidleiters 36 zwischen 0,4 mm und 1,00 mm betragen kann. Der Überzug kann beispielsweise aus Kupfer, Aluminium oder Silber bestehen. Kupfer weist einen spezifischen elektrischen Widerstand von etwa 0,01 Ω mm²/m und eine Wärmeleitfähigkeit zwischen 240 und 380 W /m K auf. Silber und Aluminium weisen hingegen einen spezifischen elektrischen Widerstand von etwa 0,016 Q mm²/m und 0,026 Q mm²/m sowie eine Wärmeleitfähigkeit von etwa 429 W /m K und 160 W /m K auf. Der Schneidleiter 36 und/oder der Rückleiter 37 besteht bzw. bestehen vorzugsweise aus Edelstahl, Inconel oder Kanthal-D, die einen vergleichsweise hohen spezifischen Widerstand von etwa 0,7 und 1,5 Ω mm²/m und eine vergleichsweise niedrige Wärmeleitfähigkeit zwischen 15 und 25 W /m K weist.

Der Isolierkörper 34 ist thermisch und elektrisch isolierend. Dies führt dazu, dass Wärme in dem zentralen Bereich zwischen den Branchen 15, 16 größtenteils nur an dem Schneidleiter 36 gehalten wird. Wärme, die hingegen im distalen Endbereich 28 der ersten Branche 15 durch Wärmeleitung in den Rückleiter 37 übergeht, wird insbesondere über den Überzug 43 des Rückleiters 37 auf diesen verteilt und kann großflächig ohne Ausbildung lokaler Hotspots abgeleitet werden. Dies ermöglicht es, die Branchen 15, 16 besonders schlank, dünn und flach auszugestalten, ohne, dass sich die Außenseiten der Branchen, insbesondere bei langem Betrieb, aufheizen.

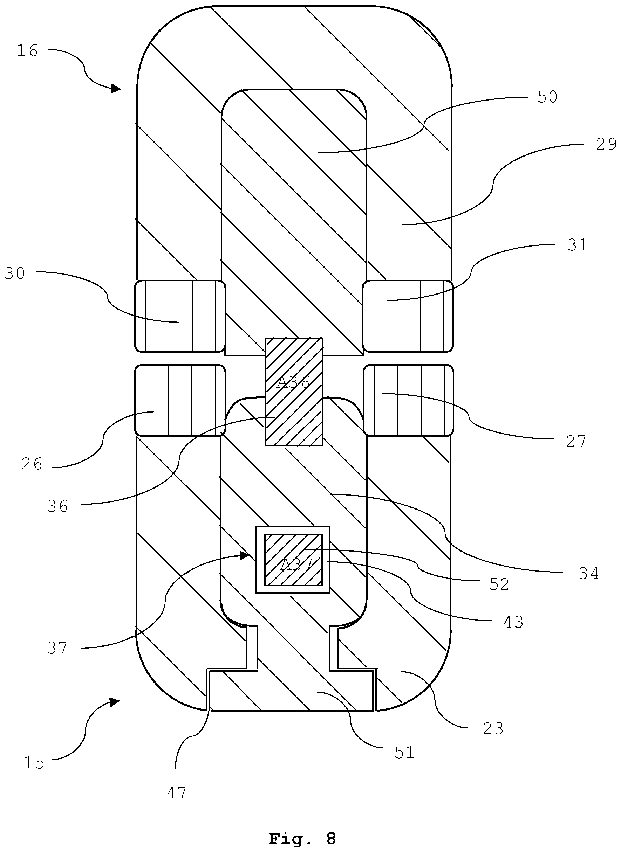

Anders als in Figur 7 dargestellt, kann das Tragteil 23 der ersten Branche 15 an seiner Unterseite um die Öffnung 46 herum Taschen 47 aufweisen, so dass die Isolierkörperfüße 51 bündig mit dem Tragteil 23 abgetrennt werden können. Dies ist in Figur 8 dargestellt.

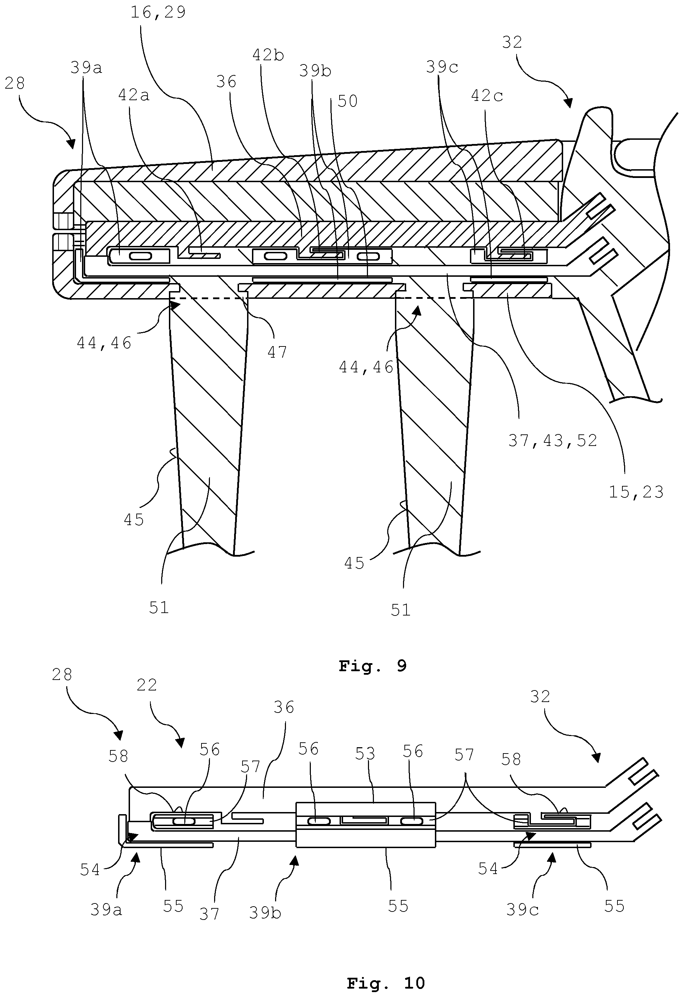

In den Figuren 9 bis 12 ist ein weiteres Beispiel für die Ausgestaltung der Branchen dargestellt. Für das in den Figuren 9 bis 12 gezeigte Beispiel gilt das zuvor Gesagte mit Bezug auf die Bezugszeichen entsprechend.

Dieses Beispiel unterscheidet sich von den vorherigen dadurch, dass die erste Branche 15 nur zwei Isolierkörperfüße 51, dafür aber drei Abstandselemente 39a, 39b, 39c, aufweist. Der Schneidleiter 36 weist verschiedene Hinterschneidungsabschnitte 42a, 42b, 42c auf, welche entweder im Isolierkörper 34 (vgl. Hinterschneidungsabschnitt 42a) oder innerhalb eines Abstandselements 39b, 39c (vgl. Hinterschneidungsabschnitte 42b, 42c) liegen können.

Die Abstandselemente 39a, 39b, 39c bilden mit den Hinterschneidungsabschnitten 42b, 42c einen Formschluss und fixieren den Schneidleiter 36, wodurch ein Abheben des Schneidleiters 36 durch Gewebeanhaftung verhindert werden kann. Die Abstandselemente 39a, 39b, 39c verhindern den elektrischen Kontakt zwischen Schneidleiter 36 und Rückleiter 37 sowie zum Tragteil 23 der ersten Branche 15.

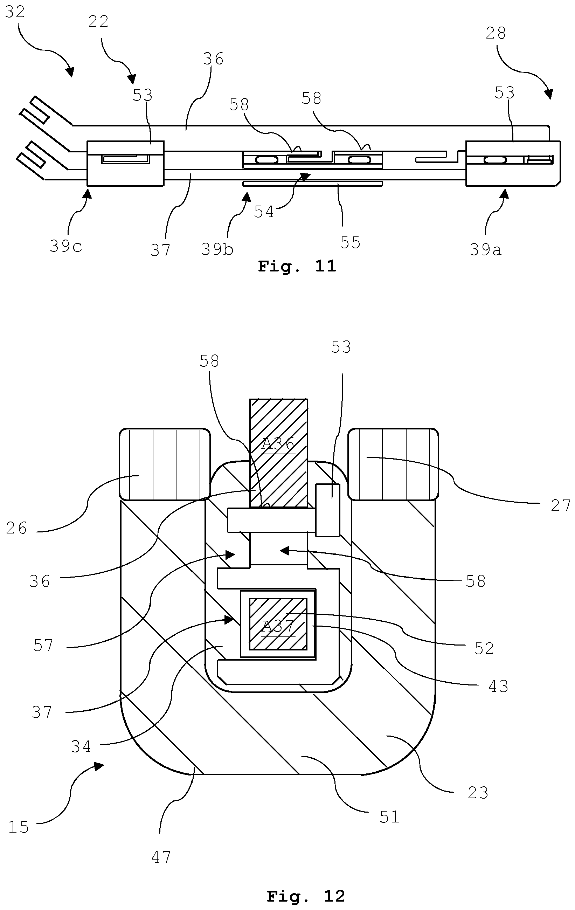

Wie in Figur 10 von einer Seite und in Figur 11 von der anderen Seite dargestellt wird, weisen die Abstandselemente 39a, 39b, 30c zudem seitliche Anschläge 53 auf, mit denen auch die mittige Positionierung des Schneidelements 22 in der ersten Branche 15 sichergestellt werden kann. Die seitlichen Anschläge 53 grenzen an eine Auflagefläche 58 seitlich an, auf der Schneidleiter 36, insbesondere seine Unterseite, aufliegt und sich abstützen kann. Die Abstandselemente 39a, 39b, 39c weisen Rückleiteraussparungen 54 auf, die jeweils zu einer Seite geöffnet sind. Die Rückleiteraussparungen 54 können diese wechselseitig auf den Schneidleiter 36 und Rückleiter 37 aufgesteckt werden, wodurch eine mittige Positionierung im Tragteil 23 der ersten Branche 15 erzielt werden kann. Das proximale Abstandselement 39c weist außerdem eine an das Tragteil 23 angepasste Bodenplatte 55 auf, die sich in Richtung des distalen Endes 28 der ersten Branche 15 hin verjüngt, so dass der Schneideleiter 36 auch im breiter werdenden proximalen scharniernahen Bereich 32 mittig positioniert werden kann.

Zusätzlich weisen die Abstandselemente 39a, 39b, 39c sowohl Quer- als auch Längsaussparungen 56, 57 auf, durch die das Material des Isolierkörpers 34 bei der Herstellung hindurchfließen kann. Es kann so ein Formschluss zwischen den Abstandselementen 39a, 39b, 39c und dem Isolierkörper 34 geschaffen werden.

Die Kombination der verschiedenen Formschlüsse - auch der Formschluss durch den Hinterschneidungsbereich 44 der Isolierkörperfüße 51 kann das Herausheben des Schneidelements 22 verhindern.

Der Werkstoff der Abstandselemente 39a, 39b, 39c kann Keramik mit elektrisch und thermisch isolierenden Eigenschaften sein, wodurch vermieden werden kann, dass sich die generierte Wärme im Schneidelement 22 verbreitet, sondern hauptsächlich von der vorgesehenen Stelle - dem Schneidleiter 36 - an das Gewebe abgegeben wird.

Die Erfindung betrifft ein elektrochirurgisches Instrument 10, insbesondere zum thermischen Schneiden von biologischem Gewebe eines Patienten. Das Instrument 10 weist mindestens zwei Branchen 15, 16 auf, wobei mindestens eine der Branchen 15 dazu eingerichtet ist, relativ zu der anderen Branche 16 auf diese zu und von dieser weg bewegt zu werden. Mindestens eine der Branchen 15 weist ein thermisches Schneidelement 22 mit einem elektrisch leitfähigen Schneidleiter 36 und einem elektrisch leitfähigen Rückleiter 37 auf, die miteinander elektrisch verbunden sind. Das Schneidelement 22 ist derart angeordnet, dass der Schneidleiter 36 an einer der anderen Branche 16 zugewandten Seite der einen Branche 15 zumindest thermisch frei liegt und der Rückleiter 37 von einem Isolierkörper 34 umgeben ist. Der Rückleiter 37 weist in Stromflussrichtung einen geringeren elektrischen Widerstand und vorzugsweise eine größere Wärmeleitfähigkeit als der Schneidleiter 36 auf. Dadurch wird an dem Rückleiter 37 bei Bestromung des Schneidelements 22 eine wesentlich geringere Wärmemenge und Temperatur als an dem Schneidleiter 36 erzeugt und der Rückleiter 37 kann zur Wärmeabfuhr verwendet werden.

### Bezugszeichenliste:

- 10: Elektrochirurgisches Instrument
- 11: Schaft
- 12: Proximales Ende des Instruments
- 13: Distales Ende des Instruments
- 14: Werkzeug
- 15: Erste Branche
- 16: Zweite Branche
- 17: Handgriff
- 18: Elektrische Zuleitung
- 19: Instrumentenzuleitung
- 20: Versorgungsgerät
- 21: Schwenkachse
- 22: Schneidelement
- 23: Tragteil der ersten Branche
- 24: Außenseite des Tragteils
- 25: Randbereich der Branchen
- 26, 27: Versiegelungselektrode der ersten Branche
- 28: Distaler Endbereich der Branchen
- 29: Tragteil der zweiten Branche
- 30, 31: Versiegelungselektrode der zweiten Branche
- 32: Scharniernaher Bereich
- 33: Nut der ersten Branche
- 34: Isolierkörper
- 35: Nut der zweiten Branche
- 36: Schneidleiter
- 37: Rückleiter
- 38: Anschlusselemente
- 39: Abstandselement
- 40: Positioniervorsprünge
- 41: Befestigungsvorsprünge
- 42: Hinterschneidungsabschnitte
- 43: Überzug
- 44: Hinterschneidungsbereich der Isolierkörperfüße
- 45: Sich verjüngender Abschnitt der Isolierkörperfüße
- 46: Öffnungen in dem Tragteil der ersten Branche
- 47: Taschen
- 48: HF-Generator
- 49: Transformator
- 50: Gegendruckkörper
- 51: Isolierkörperfüße
- 52: Kern des Rückleiters
- 53: seitlicher Anschlag
- 54: Rückleiteraussparung
- 55: Bodenplatte
- 56: Queraussparung
- 57: Längsaussparungen
- 58: Auflagefläche
- A36: Querschnittsfläche des Schneidleiters
- A37: Querschnittsfläche des Rückleiters

## Patentansprüche

1. Elektrochirurgisches Instrument (10), insbesondere zum thermischen Schneiden von biologischem Gewebe, mit mindestens zwei Branchen (15, 16), wobei mindestens eine der Branchen (15, 16) dazu eingerichtet ist, relative zu der anderen Branche (16, 15) auf diese zu und von dieser weg bewegt zu werden,
wobei mindestens eine der Branchen (15, 16) ein thermisches Schneidelement (22) mit einem elektrisch leitfähigen Schneidleiter (36) und einem elektrisch leitfähigen mit dem Schneidleiter (36) verbundenen Rückleiter (37) aufweist,
wobei das Schneidelement (22) derart angeordnet ist, dass der Schneidleiter (36) an einer der anderen Branche (16) zugewandten Seite der einen Branche (15) wenigstens teilweise zumindest thermisch freiliegt und der Rückleiter (37) von einem Isolierkörper (34) umgeben ist,
wobei der Rückleiter (37) einen geringeren elektrischen Widerstand als der Schneidleiter (36) aufweist.

2. Elektrochirurgisches Instrument (10) nach Anspruch 1, **dadurch gekennzeichnet, dass** Rückleiter (37) eine größere Wärmeleitfähigkeit als der Schneidleiter (36) aufweist.

3. Elektrochirurgisches Instrument (10) nach Anspruch 1 oder 2, **dadurch gekennzeichnet, dass** der Schneidleiter (36) und der Rückleiter (37) ein einstückiger Körper sind, der vorzugsweise zumindest im Wesentlichen U-förmig ausgestaltet ist.

4. Elektrochirurgisches Instrument (10) nach Anspruch 3, **dadurch gekennzeichnet, dass** der Schneidleiter (36) und der Rückleiter (37) in einem Abschnitt, der vorzugsweise an einem distalen Endbereich (28) der Branche (15) angeordnet ist, miteinander thermisch und elektrisch verbunden sind.

5. Elektrochirurgisches Instrument (10) nach einem der vorhergehenden Ansprüche, **dadurch gekennzeichnet, dass** der Kern des Rückleiters (52) zumindest teilweise mit einem Überzug (43) versehen ist, wobei der Überzug (43) vorzugsweise aus einem Material besteht, das einen niedrigeren spezifischen elektrischen Widerstand und/oder eine höhere spezifische Wärmeleitfähigkeit als das Grundmaterial des Schneidelements (22) aufweist.

6. Elektrochirurgisches Instrument (10) nach einem der vorhergehenden Ansprüche, **dadurch gekennzeichnet, dass** der Schneidleiter (36) eine größere Querschnittsfläche als der Rückleiter (37) aufweist.

7. Elektrochirurgisches Instrument (10) nach einem der vorhergehenden Ansprüche, **dadurch gekennzeichnet, dass** die Branche (15, 16) in einem Randbereich (25) mindestens eine Versiegelungselektrode (26, 27) aufweist, die zu dem Schneidleiter (36) beabstandet ist, wobei der Schneidleiter (36), vorzugsweise, an den Seitenflanken zwischen den distalen und proximalen Enden der Branche (15) und/oder an dem distalen Endbereich (28) der Branche (15) von der Versiegelungselektrode (26, 27) umschlossen ist.

8. Elektrochirurgisches Instrument (10) nach Anspruch 7, **dadurch gekennzeichnet, dass** die andere Branche (16) in einem Randbereich (25) mindestens eine Versiegelungselektrode (30, 31) aufweist, die identisch zu der Versiegelungselektrode (26, 27) der einen Branche (15) angeordnet ist.

9. Elektrochirurgisches Instrument (10) nach Anspruch 9, **dadurch gekennzeichnet, dass** die Versiegelungselektroden (26, 27; 30, 31) bei geschlossenen Branchen (15, 16) übereinander, aber zueinander beabstandet, angeordnet sind.

10. Elektrochirurgisches Instrument (10) nach einem der vorhergehenden Ansprüche, **dadurch gekennzeichnet, dass** der Isolierkörper (34) aus einem Material besteht, das elektrisch isolierend ist und eine niedrigere Wärmeleitfähigkeit als das Schneidelement (22) aufweist.

11. Elektrochirurgisches Instrument (10) nach einem der vorhergehenden Ansprüche, **dadurch gekennzeichnet, dass** an dem Schneidleiter (36) mindestens ein Befestigungsvorsprung (41) ausgebildet ist, der von dem Isolierkörper (34) umgeben ist, wobei der mindestens einen Befestigungsvorsprung (41) vorzugsweise einen Hinterschneidungsabschnitt (42) aufweist, der von dem Isolierkörper (34) eingeschlossen ist.

12. Elektrochirurgisches Instrument (10) nach einem der vorhergehenden Ansprüche, **dadurch gekennzeichnet, dass** zwischen dem Schneidleiter (36) und dem Rückleiter (37) wenigstens ein Abstandselement (39, 39a, 39b, 39c) angeordnet ist, das aus einem Material besteht, dass elektrisch und thermisch isolierend ist.

13. Elektrochirurgisches Instrument (10) nach einem der Anspruch 12, **dadurch gekennzeichnet, dass** das wenigstens eine Abstandselement (39) eine Oberseite und eine Unterseite aufweist, wobei das Abstandselement (39) vorzugsweise an der Oberseite an dem Schneidleiter (36) und/oder an der Unterseite an dem Rückleiter (37) anliegt.

14. Elektrochirurgisches Instrument (10) nach einem der Anspruch 12, **dadurch gekennzeichnet, dass** das wenigstens eine Abstandselement (39a, 30b, 39c) eine Rückleiteraussparung (54) aufweist, in der der Rückleiter (37) derart angeordnet ist, dass dieser von dem Abstandselement (39, 39b, 39c) zumindest teilweise umschlossen eingefasst ist.

15. Elektrochirurgisches Instrument (10) nach einem der Anspruch 14, **dadurch gekennzeichnet, dass** das wenigstens eine Abstandselement (39a, 39b, 39c) eine Auflagefläche (58) aufweist, auf der der Schneidleiter (36) aufliegt, wobei das Abstandselement (39a) wenigstens einen seitlichen Anschlag (53) aufweist, der von der Auflagefläche (58) vorsteht und an dem der Schneidleiter seitlich anliegt.

16. Elektrochirurgisches Instrument (10) nach einem der vorhergehenden Ansprüche, **dadurch gekennzeichnet, dass** die andere Branche (16) einen Gegendruckkörper (50) aufweist, der an einer der einen Branche (15) zugewandten Seite angeordnet ist und dort eine Gegendruckfläche für den Schneidleiter (36) der einen Branche (15) definiert.

17. Elektrochirurgisches Instrument (10) nach einem der vorhergehenden Ansprüche, **dadurch gekennzeichnet, dass** der Schneidleiter (36) von der durch den Isolierkörper (34) gebildeten Oberfläche in Richtung der anderen Branche (16) hin vorsteht.

18. Elektrochirurgisches Instrument (10) nach Anspruch 16 oder 17, **dadurch gekennzeichnet, dass** sich der Gegendruckkörper (50) und der Schneidleiter (36) bei geschlossenen Branchen (15, 16) entlang des Schneidleiters (36), vorzugsweise mit einer gleichmäßigen Tiefe, überschneiden, wobei der Schneidleiter (36) in den Gegendruckkörper (50) eindringt.
